# EUROPEAN PATENT APPLICATION

(11) **EP 2 335 676 A2**
(43) Date of publication of application: **22.06.2011**
(21) Application number: 11151518.5
(22) Date of filing: 25.05.2006
(51) Int. Cl.: A61K 8/19, A61Q 9/04, A61K 8/46, A61K 8/92

(54) **Depilatory compositions**

(30) Priority: 26.05.2005 US 138110
(62) Divisional of application: 06771291.9
(71) Applicant: Church & Dwight Company, Inc., Princeton, NJ 08543-5297 (US)
(72) Inventor: Adamy, Steven T., Lawrenceville, NJ 08648 (US); Lee, Andrew S., West Windsor NJ 08550 (US); Kepa, Anna, Sayreville, Nj 08872 (US)
(74) Representative: Rushton, David John

(57) **Abstract**

The present invention is directed to dual phase and single phase depilatory compositions and methods of using said compositions. More specifically, the present invention is directed to a dual phase composition for the removal of hair comprising a first acidic phase containing a thiol-based depilatory agent and a second alkaline phase containing a pH buffer. The present invention is also directed to a single phase depilatory composition comprising 2-aminoehtanethiol and a pH buffer and an advantageous packaging means for said single phase depilatory composition.

## Description

This application is a continuation-in-part of co-pending U.S. application Serial No. 11/138,110, filed May 26, 2005.

### FIELD OF THE INVENTION

The present invention relates to depilatory compositions, their preparation, and their use in degrading hair keratin.

### BACKGROUND OF THE INVENTION

Compositions for removing superfluous body hair are well known and are of various types. One type of composition requires initial heating before being applied to the skin in a generally molten state. It is then allowed to solidify before being removed from the skin together with unwanted hair.

Another type of depilatory composition is in the form of a cream, which can be applied to the skin at room temperature. The cream includes a substance that degrades hair keratin. Such substances tend to irritate the skin, which is a problem for users with sensitive skin. Compositions with reduced irritancy have been sought.

The use of thiol-based depilatory agents, such as thioglycolic acid, for removal of unwanted body and facial hair is well established in the art. These agents react by reducing hair's protein disulfide bonds to sulfhydryl anions, thereby allowing easy removal of the weakened hairs when washed or wiped away. However, in using thiols, it was discovered that certain conditions facilitated the effectiveness of this reaction. One such condition is high alkalinity to provide ionized reactants. Not only does the high pH (approximately 12.0-13) result in ionized thiols, but they also result in increased penetration of a reactant. Substances to provide further enhancement of penetration by active thiols were developed.

Chemical-based depilation offers an attractive alternative to shaving, with the removal of hair being accomplished through the cleavage of disulfide bonds in the hair fiber as well as denaturation of the associated protein matrix. However, a relatively small proportion of individuals, compared with shaving, employ depilatories. One reason for the limited use may be that the hair cleavage reaction must be run at a very high pH. A typical example is the Nair® lotion product, having a pH of about 12.5 to 13. The necessity for the high pH is due to two reasons: 1), the high pH aids in the swelling of the hair fiber due to protein denaturation, and 2), the keratin reduction proceeds through the formation of the mercaptide ion (RS⁻), which in turn cleaves the disulfide bond through a nucleophilic displacement reaction:

Ker-S-S-Ker + RS' ↔ Ker-S-S-R + Ker-S⁻

Ker-S-S-R + RS' ↔ R-S-S-R + Ker-S⁻

Depilatories are therefore often formulated upwards of pH 12 or higher. For example, Schamper (U.S. 4,121,904) cites examples at pH 11.8, 12.0, 12.2 and 12.3 (although the patent claims compositions in the range of pH 10 to 12.5). Sliwinski (U.S. 3,527,559) cites examples in the range of 12.0 to 12.5 (with claims between 11.5 and 13.5), while de la Garcia (U.S. 3,981,681) notes an example at pH 12.3 (with claims in the range of 11.5 to 12.7).

It therefore generally appears that, despite patent claims in the range of 10.5 to 13.5, most actual examples are formulated at a pH around 12. It would be beneficial to formulate an effective depilatory at a pH value that would be less damaging to skin than products at pH 12 or above. One way to increase the rate of the disulfide cleavage at a reduced pH is to employ a very reactive mercaptan, where the -SH proton is quite acidic. Unfortunately, such compounds readily react with themselves:

RS⁻ + RS⁻ ↔ 2R-S-S-R

Formation of the R-S-S-R disulfide consequently reduces product activity.

It would be most useful to formulate highly active mercaptans in such a way as to keep them in the RSH form, but be able to be employed at a higher pH in order to cleave hair. Because the mercaptan is highly reactive, it could also cleave hair at a pH lower than that of typical depilatories.

### SUMMARY OF THE INVENTION

One aspect of the present invention is directed to dual phase depilatory compositions comprising a first acidic phase containing a thiol-based depilatory agent and a second separate alkaline phase containing a pH buffer. A dual chamber mixing and dispensing system keeps the acidic and alkaline phases separated. The two phases are mixed just prior to or during application for hair removal.

Another aspect of the present invention is directed to single phase depilatory compositions comprising a 2-aminoethanethiol (2AET) depilatory agent, which is highly active and more effective for hair removal at the typical compositional pH used for depilation. As such 2AET has been formed more effective at lower pH than other known depilatory agents. The 2AET compositions are shown to remain stable and have extended shelf life if exposure to oxygen is kept to a minimum. Accordingly, the 2-aminoethanethiol is placed in a package which greatly reduces oxygen exposure during storage and use.

### BRIEF DESCRIPTION OF THE DRAWINGS

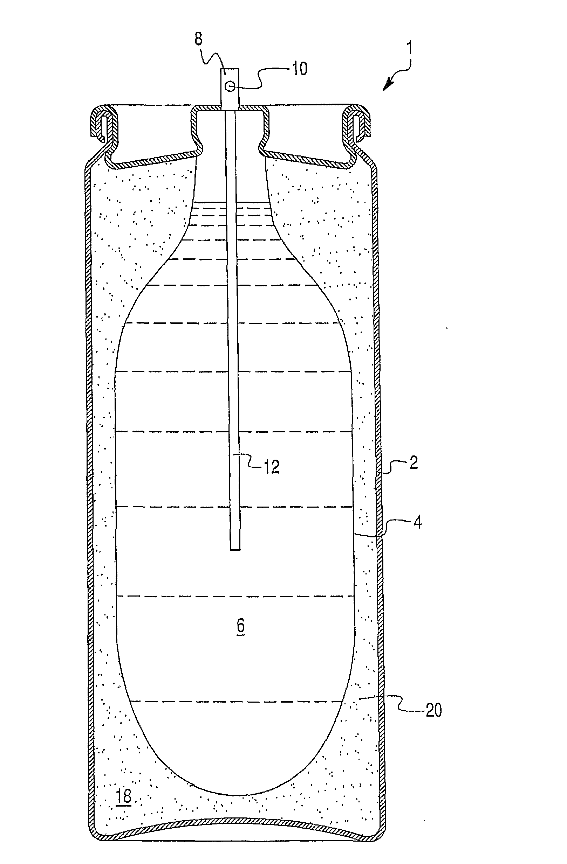

Figure 1 is a schematic of an illustrative bag-on-valve aluminum can storage system.

### DETAILED DESCRIPTION OF THE INVENTION

The present invention is based on the use of thiol-based depilatory agents for hair removal.

In one embodiment of the present invention, the depilatory composition is maintained in a dual phase system. The first phase is acidic while the second separate phase is alkaline. The two phases are kept separate and mixed immediately prior to use.

The first acidic phase contains a thiol-based depilatory agent. Due to the characteristics of the thiol-based depilatory agent, this phase maintains an acidic pH. In such an acidic environment, the thiol-based depilatory agent is stable and avoids excessive degradation.

The second phase is alkaline, containing a salt to buffer the system at a higher pH. Examples of pH buffers include calcium hydroxide and sodium hydroxide. Due to the characteristics of the pH buffer, this phase maintains an alkaline pH. An alkaline environment is highly beneficial for the hair removal process. A mixture of the two phases results in a mixed composition with a pH high enough to cause effective depilation, but low enough to minimize irritation. Furthermore, mixing the two phases results in a temperature increase due to the exothermic reaction between the acidic and basic phases. The increased temperature, i.e., the heated depilatory, increases the depilation rate.

The dual phase system employs separate vehicles for the thiol active, kept at an acidic pH, and the alkaline phase containing a pH buffer. The advantage of maintaining the two phases separate stems from the fact that thiol compounds are susceptible to dimerization at high pH, promoted by the interaction with O₂. The mechanism for the base (:B) catalyzed oxidation has been proposed (S. Oae, Organic Sulfur Chemistry: Structure and Mechanism, CRC Press, Inc., Boca Raton, 1992, p. 204).

RSH + :B ↔ RS⁻ + BH

RS⁻ + O₂ → RS• + •O₂⁻

RS⁻ + •O₂⁻ → RS• + O₂²⁻

2RS• → RSSR

2O₂²⁻ + 2RSH → O₂ + 2RS⁻ + 2OH⁻

From the mechanism above, it would seem that keeping the thiol at a lower pH may inhibit dimerization and improve stability. Base from the second part of the two-part formula could then be added at the time of use. It is also possible that a higher concentration of RS-would be available at the time of use, compared with the case if it were stored at a high pH, since at the high pH, the 2RS⁻ ↔ RSSR equilibrium will have already decreased the RS⁻ concentration. Use of a two-part depilatory as in the present invention is effective when used with a dual-chamber package. Such package would preferably contain a mixing chamber by which the first acidic phase and the separate alkaline phase could be mixed before application to the hair. Such mixing and dispensing systems for dual phase packages are known in the art.

The first acidic phase contains a thiol-based depilatory agent. The thiol-based depilatory agent includes, but is not limited to, one or more thiol acids, (e.g. thioglycolic, thiolactic acid, and β-mercaptopropionic acid), or the alkali and/or the alkaline-earth metal salts of these acids. In addition, other active thiol agents can be used. These include β-mercaptoethanol, thioglycerols, 1,3-dithio-2-propanol, 1,4-dithio-2-butanol, 1,4-dimercapto-2,3-butanediol, 1,3-dithio-2-methoxypropane, 1,3-dimercapto-2-aminopropane, 1,4-dimercapto-2,3-diaminobutane, aminoethanethiol, and related effective thiol actives.

The dual phase system further comprises a separate alkaline phase containing a pH buffer. The pH buffer includes, but is not limited to, calcium hydroxide and sodium hydroxide.

The present invention can be embodied in several commercial forms such as creams, lotions, gels, aerosols, or the like. Aqueous-based systems are particularly exemplified. Examples of such systems are illustrated below. Each phase can be formulated differently, but should be compatible to yield a uniform composition when mixed and that can be readily applied and removed by the user.

Depending on the form of the composition, a number of optional ingredients can be added to each phase. These ingredients include from about 0% to about 5% of a suitable filler such as chalk, magnesium oxide and carbonate, clays, talc, fumed silica and mixtures thereof. In one embodiment, Promulgen^{™} D (Chemron, Paso Robles, CA), which is a mixture of cetearyl alcohol and ceteareth-20 can be used as an emulsifying agent. Emulsifying agents such as anionic surfactants (e.g., fatty alcohol sulfates and/or alkyl aryl sulfates), nonionic surfactants, and mixtures thereof, present at levels of from about 0% to about 20%, are also useful. Often chelating agents to complex with metals are included in such compositions. One suitable example of such a chelator is ethylenediaminetetraacetic acid, its salts and mixtures thereof.

Among the other ingredients useful in these various embodiments is a gelling agent or thickener, present at levels of from about 0% to about 30%. The thickeners used could include both natural and synthetic ones such as tragacanth, xanthan, karaya, and guar gums, clays, methyl or hydroxyethyl cellulose, hydroxypropyl cellulose, carboxymethyl cellulose, fatty and polyvinyl alcohols, modified starches and sugars, and mixtures thereof. Emollients such as paraffin, petrolatum, mineral oil, fatty alcohols, silicone oils, and mixtures thereof present at levels of from about 0% to about 60%, can also be included. Fragrance and coloring generally provide the remaining ingredients.

Advantageously, since a dual phase composition is provided, certain additives such as fragrances, that are typically unstable at high pH, for example pH of 8 or higher, can now be included in the acidic phase. The rationale for this is that in a single phase system these ingredients are exposed to a highly alkaline environment for extended periods of time during storage prior to consumer usage causing chemical degradation, product yellowing and product odor. However, in a dual phase system these ingredients, unstable at high pH, can be held in the acidic phase in which the pH will range from about 3 to about 6.5, where such ingredients will remain stable. Once the two phases are combined, the 3-4 minute exposure time is not long enough to cause significant degradation of these alkaline sensitive materials. Thus, alkaline sensitive ingredients such as moisturizers, anti-oxidants, tanners, fragrances, anti-aging ingredients, and other like ingredients can now be included in depilatory compositions. Specifically, ingredients such as esters (for improved skin feel/moisturization properties), natural oils and/or butters (for moisturization/anti-oxidant properties), anti-aging ingredients, viscosity modifiers, sunless tanning ingredients, silicone oils (for improved skin feel/rinse fell properties), and fragrance components such as aldehydes, ketones, and esters can be added to improve the overall effectiveness and desirability of depilatory compositions.

In another embodiment of the present invention, clear systems for both the acidic and alkaline phases can be formulated. Examples 3-5 and 8, below, exhibit clear first acidic phases. Such clear acidic phase components may include, but are not limited to, surfactants and thickeners, such as ethylene oxide/propylene oxide block copolymers and hydroxypropyl cellulose polymer, respectively. However, formulating a clear system for the alkaline phase is more challenging. Potassium carbonate may be a viable alternative to alkaline phase pH buffers such as calcium hydroxide and sodium hydroxide. Preferably, a chosen alkaline salt should be soluble in water. Also, preferred thickening systems should be resilient against both a high salt concentration and a high pH.

An additional embodiment of the present invention entails one phase of the dual phase system being anhydrous. For example, 2-aminoethanethiol (2AET) is soluble in propylene glycol. If the first acidic phase is formulated in propylene glycol, and the separate alkaline phase is formulated in water, mixing the two results in a temperature increase due to the exothermic mixing between water and propylene glycol. The increased temperature should increase the depilation rate. Even in aqueous systems the acid base reaction will tend to increase the temperature of the composition.

The present invention further embraces a depilatory composition found in an active single phase system. The single phase system includes a thiol-based depilatory agent, 2AET, and a pH buffer as defined above. Additional components, such as thickeners, surfactants and conditioners may also be incorporated. An example of a conditioner is polyquaternium-7, but any conditioners commonly known in the art are acceptable. Thickeners and surfactants are defined above. One advantage of using the single phase depilatory agent of the present invention is obtaining a faster depilation rate at traditional pH levels, e.g., pH 12-13, or alternatively, similar depilation rates at lower pH levels, e.g., pH of 12 or less, both resulting in lowered skin irritation. Typically, 2AET levels in the single phase can be from about 3 % to about 10 %, by weight, preferably from about 5 % to about 7.5 %, by weight.

It has further been found that certain natural emollients such as lanolin, vegetable butters and oils can be used to smooth these single phase depilatories. Non-limiting examples include cocoa butter, shea butter, avocado oil, etc. In general, the amount of such fatty acid component in the composition may range from about 0.5-5% by weight.

Because thiols, such as 2AET, undergo dimerization at high pH, promoted by the interaction with O₂, in a single phase system the packaging should minimize the thiol component's exposure to oxygen during storage and consumer use. An embodiment for minimizing oxygen exposure is a foil laminate tube. Other known packages can be used as well. For example, the single phase depilatory can be packaged in an aluminum can with a bag-on-valve system (see **Figure 1**, described in further detail below in Example 13). The use of such packaging can improve the useful life of the single phase depilatory by reducing degradation of the active thiol-based depilatory agent.

In another embodiment, the single phase depilatory can be heated to increase depilation rate. For example, the single phase depilatory of the present invention could be packaged in a product package system, which is capable of heating the product before use. The package could be self-heating, microwaved, or heated with hot water.

### EXAMPLE 1

### Emulsion 2AET System

Two emulsion vehicles were prepared. The acidic phase contained 2-aminoethanethiol (2AET) [with no Ca(OH)₂]. The composition is shown below:

| **Part A** | | |
|---|---|---|
| | **Component** | **Wt. %** |
| | 2AET • HCl | 14.3 |
| | Deionized Water | 68.6 |

| **Part B** | | |
|---|---|---|
| | **Component** | **Wt. %** |
| | Mineral Oil | 7.6 |
| | Promulgen D (C₁₆EO₂₀/C₁₆OH) | 9.5 |

Parts A and B were prepared separately. For Part A, the 2AET • HCl was dissolved in the water at room temperature. For Part B, the mineral oil was heated to 85°C, at which point the Promulgen D was added. While reducing the temperature of Part B to 75°C, Part A was heated to 75°C. Under vigorous agitation, Part B was added to Part A. The mixture was then stirred for about 5 minutes at 75°C, and then removed from the heat. Stirring was continued as the mixture cooled. At about 40°C, the mixture was homogenized for about 1 minute. The mixture was then allowed to cool to room temperature. The pH was found to be about 5.3.

The Ca(OH)₂ phase of the emulsion-based two-part depilatory was prepared in a similar fashion. The composition is listed below:

| **Part A** | | |
|---|---|---|
| | **Component** | **Wt. %** |
| | Ca(OH)₂ | 10.0 |
| | Deionized Water | 77.0 |

| **Part B** | | |
|---|---|---|
| | **Component** | **Wt. %** |
| | Mineral Oil | 8.0 |
| | Promulgen D (C₁₆EO₂₀/C₁₆OH) | 5.0 |

The pH of the Ca(OH)₂ phase was found to be about 12.7.

The two-part emulsion system was evaluated by mixing equal parts (by mass) of the 2AET and Ca(OH)₂ phases. Mixing was generally easy with no coagulation. The final pH of the mixture was 12.0. In-vivo depilation exhibited a time of 3-4 minutes.

### EXAMPLE 2

### Emulsion 2AET System

Two emulsion vehicles were prepared. The acidic phase contained 2AET (with no Ca(OH)₂). The composition is shown below:

| **Part A** | | |
|---|---|---|
| | **Component** | **Wt. %** |
| | 2AET • HCl | 15.0 |
| | Deionized Water | 70.0 |

| **Part B** | | |
|---|---|---|
| | **Component** | **Wt. %** |
| | Mineral Oil | 8.0 |
| | Promulgen D (C₁₆EO₂₀/C₁₆OH) | 7.0 |

Parts A and B were prepared separately. For Part A, the 2AET • HCl was dissolved in the H₂0 at room temperature. For Part B, the mineral oil was heated to 85°C, at which point the Promulgen D was added. While reducing the temperature of Part B to 75°C, Part A was heated to 75°C. Under vigorous agitation, Part B was added to Part A. The mixture was then stirred for about 5 minutes at 75°C, and then removed from the heat. Stirring was continued as the mixture cooled. At about 40°C, the mixture was homogenized for about 1 minute. The mixture was then allowed to cool to room temperature. The pH was found to be about 5.0.

The Ca(OH)₂ phase of the emulsion-based two-part depilatory was prepared in a similar fashion. The composition is listed below:

| **Part A** | | |
|---|---|---|
| | **Component** | **Wt. %** |
| | Ca(OH)₂ | 10.0 |
| | Deionized Water | 77.0 |

| **Part B** | | |
|---|---|---|
| | **Component** | **Wt. %** |
| | Mineral Oil | 8.0 |
| | Promulgen D (C₁₆EO₂₀/C₁₆OH) | 5.0 |

The pH of the Ca(OH)₂ phase was found to be about 12.8.

The two-part emulsion system was evaluated by mixing equal parts (by mass) of the 2AET and Ca(OH)₂ phases. Mixing was generally easy with no coagulation. The final pH of the mixture was 12.0. In-vivo depilation exhibited a depilation time of 3-4 minutes.

The above composition was then tested in a larger scale clinical test of 26 subjects. The average depilation time for the Example 2 composition was 3.64 minutes with a SD of 0.51 minutes, which compared favorably to a commercially available product. The Example 2 composition, however, exhibited a far lower degree of irritation, with zero subjects reporting an adverse reaction.

### EXAMPLE 3

### Dual Phase 2AET System

A depilatory system, composed of two parts, is described. The first part is slightly acidic and contains a mercaptan. The second component is alkaline, containing a salt to buffer the system at a higher pH. Mixture of the two components results in a mixture with a pH high enough to cause depilation but low enough to minimize irritation.

| **Formulation of the Acidic Mercaptan Component** | |
|---|---|
| **Component** | **Wt. %** |
| 2-aminoethanethiol • HCl | 14.76 |
| Guar hydroxpropyltrimonium chloride (N-Hance 3215, from Hercules, Wilmington, DE) | 1.58 |
| Deionized Water | 83.66 |

The 2AET was first added to the water, with the pH of this solution found to be about 4.5. Under agitation, the N-Hance 3215 was then slowly added. The resulting mixture had a pH of about 6.1 and was in the form of very thick syrup.

| **Formulation of the Alkaline Component** | | |
|---|---|---|
| | **Component** | **Wt. %** |
| | Polyvinyl alcohol (87-89% hydrolyzed, | 1.74 |
| | MW=11,000 -13,000) | |
| | Sodium magnesium lithium silicate | 4.35 |
| | Ca(OH)₂ | 8.69 |
| | Deionized Water | 85.22 |

The water was heated to 75° C. The polyvinyl alcohol was then added under good agitation. Upon dissolution of the polyvinyl alcohol, the sodium magnesium lithium silicate was added and the mixture was stirred. Upon formation of the gel, the mixture was removed from the heat. The Ca(OH)₂ was then dispersed into the gel. The mixture thinned somewhat upon addition of the Ca(OH)₂, but still formed a creamy paste. The paste was then homogenized with a hand-held homogenizer.

Equal amounts (by mass) were mixed together until uniform. The pH of the resulting mix was about 11.3. This pH was therefore lower than typical depilatory lotions (pH 12-13). The mixture was applied to a section of leg hair. A paper towel was used to remove sections of the covered area at time intervals of 1 minute up to a total time of 5 minutes. The depilatory time was estimated to be between 3 and 5 minutes

### EXAMPLE 4

### Pluronic Gel 2AET System

A second pair of systems, based on gels made with Pluronic F127 (ethoxy-propoxy block copolymer from BASF) was evaluated. The acidic phase was composed of the following:

| **Component** | **Wt. %** |
|---|---|
| 2AET • HCl | 12.3 |
| Pluronic F127 | 18.0 |
| Deionized Water | 69.7 |

The water was heated to 85°C, at which time the F127 was added under agitation. After the F127 was fully dissolved the 2AET • HCl was added. While the 2AET • HCl was dissolving, the mixture was removed from the heat and allowed to cool. The mixture formed a clear, ringing gel at temperatures approximately below 60°C. The pH of the gel was about 5.5.

The alkaline side of the depilatory contained the following:

| **Component** | **Wt. %** |
|---|---|
| Ca(OH)₂ | 7.8 |
| Pluronic F127 | 18.9 |
| Deionized Water | 73.3 |

The alkaline phase was prepared in a manner similar to that of the acidic phase. The pH of the alkaline phase was about 12.7.

The two-part F127 gel system was evaluated by mixing equal parts (by mass) of the 2AET and the Ca(OH)₂ phases. Mixing was generally easy with no coagulation. The final pH of the mixture was 12.0. In-vivo depilation exhibited a time of 3-4 minutes.

### EXAMPLE 5

### Pluronic Gel/Silicate 2AET System

A depilation test of a mixture made with equal parts (by volume) of the F127-based 2AET gel (acidic phase from Example 4) and Silicate C (54% Na-silicate, from PQ, pH = 12.5) was also performed. Mixing the two materials resulted in a slight inhomogeneity and a drop in viscosity, with a resulting pH of 11.7. The depilation time was about 6-8 minutes.

### EXAMPLE 6

### Emulsion Systems with Other Thiols

The following thiol phase compositions were prepared in the manner cited in Example 2. Composition "C" is the same as that in Example 2, but is reproduced here for comparison:

| | **A** | **B** | **C** |
|---|---|---|---|
| | **(Wt. %)** | **(Wt. %)** | **(Wt. %)** |
| Potassium thioglycolate | 23.0 | | |
| (43% active solution) (KTG) | | | |
| 3-mercapto-1,2-propanediol | | 10.6 | |
| (thioglycerol) (3MPD) | | | |
| 2AET • HCl | | | 15.0 |
| Mineral Oil | 8.0 | 8.0 | 8.0 |
| Promulgen D (C₁₆EO₂₀/C₁₆OH) | 7.0 | 7.0 | 7.0 |
| Deionized Water | 62.0 | 74.4 | 70.0 |
| pH with no adjustment | 6.3 | 6.2 | 5.0 |

Compositions A, B and C were mixed 1/1 on a weight basis with the alkaline phase from Example 2. Results are compared below (the results of the 2AET system are noted again for comparison):

| **System** | **Depilation Time (Average ± SD)** | **Number of Subjects Noting Irritation 10 Minutes Following Depilation** |
|---|---|---|
| A (dual phase with KTG) | 4.50 ± 0.53 | 2/26 |
| B (dual phase with 3MPD) | 5.03 ± 1.07 | 1/26 |
| C (dual phase with 2AET) | 3.64 ± 0.51 | 0/26 |

### EXAMPLE 7

### Xanthan Gum / HEC system with 2AET

The following acidic thiol phase was prepared:

| **Material** | **Wt. %** |
|---|---|
| Deionized Water | 81.0 |
| Propylene Glycol | 1.4 |
| 2AET • HCl | 15.0 |
| Kelzan T (Xanthan Gum from Kelco) | 2.6 |

Propylene glycol was mixed into the water, followed by the 2AET • HCl. The Kelzan T was then added slowly while stirring to create a good vortex. The resulting gel had a pH of 4.9.

The alkaline phase had the following composition:

| **Material** | **Wt. %** |
|---|---|
| Deionized Water | 84.5 |
| Propylene Glycol | 0.8 |
| 40% NaOH solution in water (wt./wt.) | 2.7 |
| Natrasol HR CS (Hydroxyethyl cellulose | 2.0 |
| from Hercules) | |
| Ca(OH)₂ | 10.0 |

Propylene glycol was mixed into the water, followed by the NaOH solution. Under good agitation, the Natrasol was slowly added. Following swelling of the Natrasol, the Ca(OH)₂ was dispersed into the mixture. The pH of the dispersion was 13.2

Depilation time for the Example 7 system was 3 to 4 minutes.

### EXAMPLE 8

Separating active and alkaline phases has been shown to enhance thiol stability in cases of highly active thiols, like 2AET. The following example demonstrates this point.

Compositions were made based on three different thiols - potassium thioglycolate (KTG), 3-mercapto-1,2-propanediol (3MPD), and 2-aminoethanethiol (2AET). Compositions were made at high pH values, around 12-13, and lower values, around 4.5-7. Specific compositions are listed below:

| **Components (wt.%)** | **KTG High** | **KTG Low** | **3MPD High** | **3MPD Low** | **2AET High** | **2AET Low** |
|---|---|---|---|---|---|---|
| KTG (43% solution) | 11.5 | 23.0 | | | | |
| 3MPD | | | 5.3 | 10.6 | | |
| 2AET•HCl | | | | | 7.5 | 15.0 |
| Mineral Oil | 8.0 | 8.0 | 8.0 | 8.0 | 8.0 | 8.0 |
| Ca(OH)₂ | 5.0 | | 5.0 | | 5.0 | |
| NaOH | 1.5 | | 1.5 | | 1.5 | |
| Promulgen D | 4.5 | 7.0 | 6.5 | 7.0 | 6.5 | 7.0 |
| DI water | 69.5 | 62.0 | 73.7 | 74.7 | 71.5 | 70.0 |
| pH | 12.8 | 6.1 | 12.4 | 6.2 | 11.8 | 4.6 |

Compositions at alkaline pH values represent "single phase" formulas while acidic formulas represented the acidic active phase of a dual phase system.

The emulsion samples were then placed in 4 ounce HDPE bottles with screw tops. In one series of samples, the bottles were filled completely (100g), and in another series, the bottles were filled half-way (50g). The samples were set up for aging at 40°C (104°F).

Stability results of thiol degradation after 12 weeks were obtained. Shown below are results of % thiol remaining in the full and half-full HDPE bottles:

| **Percentage of RSH remaining, full HDPE bottles** | | | | | | |
|---|---|---|---|---|---|---|
| Time | KTG | KTG | 3MPD | 3MPD | 2AET | 2AET |
| (weeks) | pH 12.8 | pH 6.1 | pH 12.4 | pH 6.2 | pH 11.8 | pH 4.6 |
| 0 | 100.0 | 100.0 | 100.0 | 100.0 | 100.0 | 100.0 |
| 4 | 96.3 | 98.0 | 96.2 | 98.1 | 98.6 | 98.6 |
| 8 | 96.3 | 96.0 | 90.6 | 95.2 | 94.4 | 97.3 |
| 12 | 94.4 | 96.0 | 92.5 | 94.3 | 90.3 | 95.9 |

| **Percentage of RSH remaining, half-full HDPE bottles** | | | | | | |
|---|---|---|---|---|---|---|
| Time | KTG | KTG | 3MPD | 3MPD | 2AET | 2AET |
| (weeks) | pH 12.8 | pH 6.1 | pH 12.4 | pH 6.2 | pH 11.8 | pH 4.6 |
| 0 | 100.0 | 100.0 | 100.0 | 100.0 | 100.0 | 100.0 |
| 4 | 92.6 | 94.0 | 92.5 | 94.3 | 86.1 | 97.3 |
| 8 | 92.6 | 90.0 | 88.7 | 87.6 | 79.2 | 95.2 |
| 12 | 88.9 | 82.0 | 83.0 | 87.6 | 72.2 | 93.2 |

The greatest degree of thiol degradation occurred in the 2AET system in the half-full bottle at pH 11.8, with a reduction in the activity of about 28%. Reduction in the half-full 2AET bottle at the reduced pH was only about 7%.

Interestingly, the reduction in the full bottle at the high pH was only about 10%. The ability of the full bottle to maintain a fairly high activity implies that a single phase system incorporating 2AET at a high pH may have an acceptable shelf stability. Degradation after opening may be reduced by appropriate packaging.

### EXAMPLE 9

### Dual Phase Thioglycolic Acid System

A depilatory system, comprising of two phases, is described. The first phase being slightly acidic and the second being alkaline, containing a salt to buffer the system at a higher pH. The composition is shown below:

| **Components** | **Phase 1** | **Phase 2** |
|---|---|---|
| Water | QS | QS |
| Thioglycolic acid | 7.14% | 0% |
| Cetearyl alcohol and | | |
| ceteareth-20 | 5% | 5% |
| Mineral oil | 8% | 8% |
| Lanolin | 1% | 1% |
| Sodium hydroxide | 0% | 3% |
| Calcium hydroxide | 0% | 8% |
| Potassium hydroxide (86%) | 0% | 4.98% |

Phase 1 and 2 were prepared separately. Both phases were prepared by the following directions: First thioglycolic acid or sodium hydroxide, calcium hydroxide, and potassium hydroxide, respectively, were added as required to water at room temperature. The samples were mixed for 5 minutes. After mixing the samples were heated to 75° C and cetearyl alcohol and ceteareth-20 were added and the samples mixed for an additional 5 minutes. After which time mineral oil and lanolin were added and mixed into the samples for 5 minutes. The samples were then cooled to 50° C and homogenized for two minutes. The samples were continuously mixed while cooling to 35° C, at which time the process was stopped.

The two phase depilatory was evaluated by mixing equal parts (by mass) of the two phases. Upon mixing the temperature of the two phase system was raised from 22° C for the individual phases to 30° C for the mixture. This increase in temperature for the depilatory composition results in an increase in product efficacy.

### EXAMPLE 10

### Dual Phase 2AET System

A depilatory system, comprising of two phases, is described. The first phase being slightly acidic and the second being alkaline, containing a salt to buffer the system at a higher pH. The composition is shown below:

| **Component** | **Phase 1** | **Phase 2** |
|---|---|---|
| Water | QS | QS |
| 2AET | 15.00% | 0% |
| Cetearyl alcohol and | | |
| ceteareth-20 | 6.0% | 6.0% |
| Mineral oil | 8% | 8% |
| Sodium hydroxide | 0% | 9.8% |
| Calcium hydroxide | 0% | 10% |

Phase 1 and 2 were prepared separately. Both phases were prepared by the following directions: First thioglycolic acid or sodium hydroxide, calcium hydroxide, and potassium hydroxide, respectively, were added as required to water at room temperature. The samples were mixed for 5 minutes. After mixing the samples were heated to 75° C and cetearyl alcohol and ceteareth-20 were added and the samples mixed for an additional 5 minutes. After which time mineral oil was added and mixed into the samples for 5 minutes. The samples were then cooled to 50° C and homogenized for two minutes. The samples were continuously mixed while cooling to 35° C, at which time the process was stopped.

The two phase depilatory was evaluated by mixing equal parts (by mass) of the two phases. Upon mixing the temperature of the two phase system was raised from 22° C for the individual phases to 29° C for the mixture. This increase in temperature for the depilatory composition results in an increase in product efficacy.

### EXAMPLE 11

### Dual Phase Product Containing Benzoate Ester

A depilatory system, comprising of two phases, is described. The first phase being slightly acidic and the second being alkaline, containing a salt to buffer the system at a higher pH. In this example phase 2 contains an alkyl benzoate ester, a commonly used ingredient to deliver excellent skin feel. The composition is shown below:

| **Component** | **Phase 1** | **Phase 2** |
|---|---|---|
| Water | QS | QS |
| Thioglycolic acid | 7.14% | 0% |
| Cetearyl alcohol and | | |
| Ceteareth-20 | 4.5% | 4.5% |
| Mineral oil | 4% | 4% |
| C12-C15 alkyl Benzoate | 8% | 0% |
| Sodium hydroxide | 0% | 3% |
| Calcium hydroxide | 0% | 10% |
| Potassium hydroxide (86%) | 0% | 4.98% |

Phase 1 and 2 are prepared separately. Both phases are prepared by the following directions: First thioglycolic acid or sodium hydroxide, calcium hydroxide, and potassium hydroxide, respectively, are added as required to water at room temperature. The samples are then mixed for 5 minutes. After mixing, the samples are heated to 75° C and cetearyl alcohol and ceteareth-20 are added and the samples mixed for an additional 5 minutes. After which time mineral oil and C12-C15 alkyl benzoate are added and mixed into the samples for 5 minutes. The samples are cooled to 50° C and homogenized for two minutes. The samples are continuously mixed while cooling to 35° C, at which time the process is stopped.

The two phase depilatory is evaluated by mixing equal parts (by mass) of the two phases. Upon mixing the temperature of the two phase system is expected to raise from 22° C for the individual phases to about 30° C for the mixture. This increase in temperature for the depilatory composition results in an increase in product efficacy. Furthermore, no yellowing due to the degradation of the alkylbenzoate is expected at 40° C for at least 1 month.

### EXAMPLE 12

### Single Phase Depilatories

The following formulations were made and clinically tested to assess the depilation effectiveness and for irritation potential.

| **Component** | **Formula 1 (2AET)** | **Formula 2 (2AET)** | **Formula 3 (Potassium Thio.)** |
|---|---|---|---|
| Water | QS | QS | QS |
| Potassium | | | |
| Thioglycolate | 0% | 0% | 11.5% |
| 2AET | 7.5% | 5% | 0% |
| Ctearyl alcohol/ | | | |
| Ceteareth-20 | 6% | 6% | 6% |
| Mineral oil | 8% | 8% | 8% |
| Sodium hydroxide | 4.9% | 0% | 1.5% |
| Calcium hydroxide | 5% | 5% | 5% |

The single phase depilatory was prepared by the following directions: First thioglycolate or sodium hydroxide, calcium hydroxide, and potassium hydroxide, respectively, were added as required to water at room temperature. The sample was then mixed for 5 minutes. After mixing the sample was heated to 75° C and cetearyl alcohol and ceteareth-20 were added and the sample was mixed for an additional 5 minutes. After which time mineral oil was added and mixed into the samples for 5 minutes. The sample was then cooled to 50° C and homogenized for two minutes. The sample was continuously mixed while cooling to 35° C, at which time the process was stopped.

Formula 1 resulted in a pH of 12.42, formula 2 had a pH of 11.7, and formula 3 had a pH of 12.8. In clinical testing, formula 1 completely removed hair in an average time of 3 minutes, formula 2 completely removed hair in an average time of 3 minutes and 45 seconds, and formula 3 completely removed hair in 4 minutes; indicating that the 2AET formulas can remove hair faster or as fast at lower pH. Formulas 1 and 3 had similar irritation profiles showing mild and transient irritation while formula 2 showed no signs of irritation.

In a separate assay on forearms, formulas 2 and 3 were compared for mildness by applying the product and leaving on for 10 minutes before removal. Formula 2 resulted in only 1 out of 21 panelist showing mild transient irritation, while formula 3 showed signs of irritation in 7 out of 21 panelists.

### EXAMPLE 13

### Packaged Single Phase Depilatory

The following single phase depilatory composition was prepared:

| **Component** | **Wt %** |
|---|---|
| Water | QS |
| 2AET | 7.5% |
| Cetearyl alcohol/ | |
| Ceteareth-20 | 6% |
| Mineral oil | 8% |
| Sodium hydroxide | 4.9% |
| Calcium hydroxide | 5% |

The single phase depilatory was prepared by the following directions: First thioglycolate and sodium hydroxide, calcium hydroxide, and potassium hydroxide were optionally added as required to water at room temperature. The sample was then mixed for 5 minutes. After mixing the sample was heated to 75° C and cetearyl alcohol and ceteareth-20 were added and the sample was mixed for an additional 5 minutes. After which time mineral oil was added and mixed into the samples for 5 minutes. The sample was then cooled to 50° C and homogenized for two minutes. The sample was continuously mixed while cooling to 35° C, at which time the process was stopped.

The single phase depilatory was then packaged into two separate containers. The first was an aluminum can with a bag-on-valve system, while the second was a standard high density polyethylene bottle.

The bag-on-valve aluminum can, which is commonly known in the art, is schematically depicted in Figure 1. Referring to Figure 1, a bag-on-valve aluminum can 1 comprises an aluminum can 2, an inner plastic or flexible metallic bag 4 filled with a single phase depilatory 6 and a valve system 8, which has an outlet opening 10 through which the single phase depilatory can flow. Upon depressing the valve system 8 the single phase depilatory 6 flows through the valve system 8 and out the outlet opening 10. The inner space 18 between the aluminum can 2 and the inner bag 4 is filled with a pressurized gas 20, for example compressed air or nitrogen. When the valve system 8 is opened via depressing the valve system 8, there is provided an open flow channel through dipstick 12 and opening 10. Simultaneously, the pressurized gas 18 squeezes the bag 4 from the outside of bag 4 providing the force necessary to propel the single phase depilatory 6 up through the dipstick 12 and out opening 10. The bag-on-valve aluminum can 1 keeps air out of the inner bag 4 by preventing formation of a void area therein, which might otherwise be formed by the removal of the single phase depilatory 6 from the bag 4. By maintaining the bag 4 full at all times and reducing the ability of air to enter bag 4, provides an advantageous environment for long term stability and storage of the single phase depilatory 6.

The 2AET levels were evaluated at initial, 1 month and 3 months after storage at 40° C to determine stability profiles. The following results were obtained:

| **2AET level** | **2AET in Aluminum** | **2AET in HDPE (full)** | **2AET in HDPE (half)** |
|---|---|---|---|
| Initial | 7.3% | 7.2% | 7.1% |
| 1 month/40°C | 7.3% | 6.7% | 6.3% |
| 3 months/40° C | 7.3% | 6.6% | 5.9% |

These results indicated the usefulness of an aluminum can with a bag-on-valve system for the storage stability of active 2AET. Furthermore, even though the use of a standard high polyethylene bottle may provide a slightly less stable storage device for 2AET, the device still proves useful for improving stability during storage.

### EXAMPLE 14

### Single Phase Systems

The following compositions were prepared:

| **Material** | **Example A (Wt. %)** | **Example B (Wt. %)** |
|---|---|---|
| Deionized Water | 75.6 | 73.8 |
| 2AET • HCl | 7.4 | 7.5 |
| Mackernium 007 (Polyquaternium 7, 9% | 2.0 | 2.0 |
| Active solution from McIntyre) | | |
| Kelzan T (Xanthan Gum from Kelco) | 2.5 | 2.5 |
| 40% NaOH in water (wt/wt) | 12.5 | 14.2 |

Mackernium 007 was first mixed into the water. The 2AET • HCl was then dissolved in the water. Kelzan T was added under good agitation and dispersed. While the Kelzan T underwent swelling, the NaOH solution was added. The mixture was stirred until fully gelled. The pH of Example A was 12.1 and the pH of Example B was 12.8.

Depilation times for complete hair removal were measured at 2.5 to 3 minutes for Example A and 2 to 2.5 minutes for Example B.

### EXAMPLE 15

This experiment is a comparison of the single phase depilatory agent of the present invention (see composition 4) with other single phase depilatory agents containing 2-aminoethanethiol (2AET) and/or thioglycolic acid and biguanide and/or aminoguanidine sulfate of the prior art, including U.S. 4,631,064 and 4,618,344. The 2AET levels in compositions 4-6 were at equimolar concentration compared to the levels of 2AET/thioglycolic acid in compositions 1-3. Six total formulations were prepared. The specific compositions were:

| Materials (WT %) | 1 | 2 | 3 | 4 | 5 | 6 |
|---|---|---|---|---|---|---|
| Water | 70.8618 | 57.118 | 58.5618 | 70.2688 | 56.5188 | 57.9688 |
| 2AET-HCL | 5.1 | 5.1 | 5.1 | 8.223 | 8.223 | 8.223 |
| Thioglycolic acid | 2.53 | 2.53 | 2.53 | | | |
| Biguanide-HCL | | 13.75 | | | 13.75 | |
| Amino guanidine sulfate | | | 12.30 | | | 12.30 |
| Ca(OH)₂ | 5.00 | 5.00 | 5.00 | 5.00 | 5.00 | 5.00 |
| NaOH | 1.50 | 1.50 | 1.50 | 1.50 | 1.50 | 1.50 |
| Promulgen D | 6.5 | 6.5 | 6.5 | 6.5 | 6.5 | 6.5 |
| Mineral oil | 8.00 | 8.00 | 8.00 | 8.00 | 8.00 | 8.00 |
| Red iron oxide | 0.0075 | 0.0075 | 0.0075 | 0.0075 | 0.0075 | 0.0075 |
| Yellow iron oxide | 0.00075 | 0.00075 | 0.00075 | 0.00075 | 0.00075 | 0.00075 |
| Perfume 0/714437 | 0.50 | 0.50 | 0.50 | 0.50 | 0.50 | 0.50 |

All samples were adjusted to a pH of 11.8 by addition of 50 % NaOH. The amount of NaOH solution added was less than 2 wt %.

The compositions were tested in vivo by applying the formulation to a leg and wiping away the treatment with a paper towel at 1-minute intervals. The depilatory time was estimated to be the time at which the hair was completely removed. Depilatory times for these compositions were determined to be:

| Composition | Depilation time range (minutes) |
|---|---|
| 1 (2AET + TG) | 3-4 |
| 2 (2AET + TG + biguanide) | 5-6^{a} |
| 3 (2AET + TG + aminoguanidine sulfate) | 4-5 |
| 4 (2 AET only) | 3-4 |
| 5 (2AET + biguanide) | 3-4 |
| 6 (2AET + aminoguanidine sulfate) | 3-4 |

| | |
|---|---|
| ^{a} some stubble observed at this time interval | |

The in vivo data suggested that the addition of either biguanide or aminoguanidine sulfate did not help promote depilation. Furthermore, trends in the data also suggested that use of 2AET only instead of the 2AET/TG mixture improved depilatory activity when biguanide or aminoguanidine sulfate was present.

### EXAMPLE 16

A particularly useful single-phase depilatory composition is as follows:

| **Component** | **Wt %** |
|---|---|
| Water | QS |
| 2AET | 7.5% |
| Ceterayl alcohol/ | |
| Ceteareth-20 | 6% |
| Mineral oil | 8% |
| Lanolin | 1% |
| Sodium hydroxide | 4.9% |
| Calcium hydroxide | 5% |

This Example is equivalent to Example 13 except that a portion of the water has been replaced with lanolin to ensure solubility of any calcium ions and prevent crystallization thereof.

For the avoidance of doubt, the present application extends to the subject-matter in the following numbered paragraphs (1 to 35):
1. A dual phase depilatory composition, comprising:
   a first acidic phase, containing a thiol-based depilatory agent; and
   a separate alkaline phase, wherein said first acidic phase and said separate alkaline phase are kept apart until use.
2. The dual phase depilatory composition of paragraph 1, wherein said first acidic phase and said separate alkaline phase composition when combined has a pH of 12 or less.
3. The dual phase depilatory composition of paragraph 1, wherein said thiol-based depilatory agent is selected from the group consisting of one or more thiol acids selected from thioglycolic, thiolactic acid, β-mercaptopropionic acid, the alkali and/or the alkaline-earth metal salts of these acids, β-mercaptoethanol, thioglycerols, 1,3-dithio-2-propanol, 1,4-dithio-2-butanol, 1,4-dimercapto-2,3-butanediol, 1,3-dithio-2-methoxypropane, 1,3-dimercapto-2-aminopropane, 1,4-dimercapto-2,3-diaminobutane, and aminoethanethiol.
4. The dual phase depilatory composition of paragraph 1, wherein said first acidic phase further comprises an additive, which decomposes at a pH of above 8.0.
5. The dual phase depilatory composition of paragraph 4, wherein said first acidic phase further comprises a fragrance.
6. The dual phase depilatory composition of paragraph 5, wherein said first acidic phase further comprises a fragrance selected from the group consisting of aldehydes, ketones, and esters.
7. The dual phase depilatory composition of paragraph 4, wherein said first acidic phase further comprises one or more moisturizers, anti-oxidants, tanners, or anti-aging ingredients.
8. The dual phase depilatory composition of paragraph 1, wherein said separate alkaline phase comprises a pH buffer selected from the group consisting of calcium hydroxide and sodium hydroxide.
9. The dual phase depilatory composition of paragraph 1, wherein said thiol-based depilatory agent is potassium thioglycolate, 3-mercapto-1,2-propanediol , and 2-amino ethanethiol.
10. The dual phase depilatory composition of paragraph 1, wherein said composition comprises a heated depilatory upon mixing of said first acidic phase and said separate alkaline phase.
11. A single phase depilatory composition, comprising:
   a thiol-based depilatory agent, wherein said thiol-based depilatory agent consists essentially of 2-aminoethanethiol, said composition further containing an emulsifying agent, and a thickener;
   a pH buffer, to provide pH of at least 11.0; and
      wherein said composition is devoid of biguanide or aminoguanidine sulfate.
12. The single phase depilatory composition of paragraph 11, wherein said pH buffer is selected from the group consisting of calcium hydroxide and sodium hydroxide.
13. The single phase depilatory agent of paragraph 11, wherein said composition is packaged in a bag-on-valve aluminum can packaging system to maintain an environment favorable to product stability.
14. A single phase depilatory composition, comprising:
   3 % to 10 % by weight 2-aminoethanethiol; and
      a pH buffer, to provide said composition with a pH of less than 12.7, wherein said pH buffer comprises less than 6.5 % by weight of the composition.
15. The single phase depilatory composition of paragraph 14, wherein said pH buffer provides said composition with a pH of less than 12.0.
16. The single phase depilatory composition of paragraph 14, wherein said composition comprises 4 % to 7.5 % by weight said 2-aminoethanethiol and 4 % to 6 % by weight said pH buffer.
17. The single phase depilatory composition of paragraph 14, wherein said pH buffer is selected from the group consisting of calcium hydroxide, sodium hydroxide, and the combination thereof.
18. The single phase depilatory agent of paragraph 14, wherein said composition is packaged in a bag-on-valve aluminum can packaging system to maintain an environment favorable to product stability.
19. A single phase depilatory composition, comprising:
   2-aminoethanethiol; and
   a pH buffer, to provide said composition with a pH of less than 12.7; and
      wherein said composition provides for the complete removal of hair in 3 minutes or less.
20. The single phase depilatory of paragraph 19, wherein said composition comprises 3% to 10 % by weight 2-aminoethanethiol.
21. The single phase depilatory composition of paragraph 19, wherein said pH buffer is selected from the group consisting of calcium hydroxide, sodium hydroxide, and the combination thereof.
22. The single phase depilatory agent of paragraph 19, wherein said composition is packaged in a bag-on-valve aluminum can packaging system to maintain an environment favorable to product stability.
23. A method of hair removal, comprising the steps of:
   contacting an area of skin from which to remove hair with a depilatory composition comprising:
      2-aminoethanethiol; and
         a pH buffer, to provide said composition with a pH of less than 12.7; and
   removing said depilatory composition from contact with skin in 3
   minutes or less,
      wherein said method results in complete removal of hair.
24. The method of hair removal of paragraph 23, wherein said composition comprises 3% to 10 % by weight 2-aminoethanethiol.
25. The method of hair removal from paragraph 23, wherein said pH buffer is selected from the group consisting of calcium hydroxide, sodium hydroxide, and the combination thereof.
26. The method of hair removal from paragraph 23, wherein said composition is packaged in a bag-on-valve aluminum can packaging system to maintain an environment favorable to product stability.
27. A method of hair removal, comprising the steps of:
   contacting an area of skin from which to remove hair with a dual phase depilatory composition comprising;
      a first acidic phase, containing a thiol-based depilatory agent; and
      a separate alkaline phase, containing a pH buffer to, provide a pH of at least 1 1.0,when said first acidic phase and said separate alkaline phase are mixed;
   mixing said first and said second phases, and applying said mixture to skin; and
   removing said depilatory composition from contact with the skin after a predetermined period of time.
28. The method of hair removal from paragraph 27, wherein said thiol-based depilatory agent is selected from the group consisting of one or more thiol acids selected from thioglycolic, thiolactic acid, β-mercaptopropionic acid, the alkali and/or the alkaline-earth metal salts of these acids, β-mercaptoethanol, thioglycerols, 1,3-dithio-2-propanol, 1,4-dithio-2-butanol, 1,4-dimercapto-2,3-butanediol, 1,3-dithio-2-methoxypropane, 1,3-dimercapto-2-aminopropane, 1,4-dimercapto-2,3-diaminobutane, aminoethanethiol, and related effective thiol actives.
29. The method of hair removal from paragraph 27, wherein said pH buffer is selected from the group consisting of calcium hydroxide, sodium hydroxide, and and the combination thereof.
30. The method of paragraph 27, wherein said first acidic phase further comprises an additive, which decomposes at a pH of above 8.0.
31. The method of paragraph 30, wherein said first acidic phase further comprises a fragrance.
32. The method of paragraph 31, wherein said first acidic phase further comprises a fragrance selected from the group consisting of aldehydes, ketones, and esters.
33. The single-phase depilatory composition of paragraph 11, further including lanolin or a long chain fatty acid.
34. The single-phase depilatory composition of paragraph 14, further including lanolin or a long chain fatty acid.
35. The method of hair removal from paragraph 23, wherein said depilatory composition further includes lanolin or a long chain fatty acid.

## Claims

1. A single phase depilatory composition, comprising:
3% to 10% by weight 2-aminoethanethiol; and
a pH buffer, to provide said composition with a pH of less than 12.7, wherein said pH buffer is calcium hydroxide, wherein said calcium hydroxide buffer comprises less than 6.5% by weight of the composition.

2. The single phase depilatory composition of claim 1, wherein said pH buffer provides said composition with a pH of less than 12.0.

3. The single phase depilatory composition of claim 1, wherein said composition comprises 4% to 7.5% by weight said 2-aminoethanethiol and 4% to 6% by weight said pH buffer.

4. The single phase depilatory composition of claim 1, wherein said composition is packaged in a bag-on-valve aluminum can packaging system to maintain an environment favorable to product stability.

5. The single-phase depilatory composition of claim 1, further including lanolin or a long chain fatty acid.

6. The single phase depilatory composition of any one of claims 1 or 4 wherein said composition provides for the complete removal of hair in 3 minutes or less.

7. A method of hair removal, comprising the steps of:
contacting an area of skin from which to remove hair with a depilatory composition comprising:
2-aminoethanethiol; and
a pH buffer comprising less than 6.5% by weight of the composition,
wherein said pH buffer is calcium hydroxide, to provide said composition with a pH of less than 12.7; and
removing said depilatory composition from contact with skin in 3
minutes or less,
wherein said method results in complete removal of hair.

8. The method of hair removal of claim 7, wherein said composition comprises 3% to 10% by weight 2-ammoethanethiol.

9. The method of hair removal of claim 7, wherein said composition is packaged in a bag-on-valve aluminum can packaging system to maintain an environment favorable to product stability.

10. The method of hair removal of claim 7, wherein said depilatory composition further includes lanolin or a long chain fatty acid.
